## Europäisches Patentamt

## European Patent Office

## Office européen des brevets

(19)

(11) Numéro de publication: **0 193 428**
**B1**

(12) **FASCICULE DE BREVET EUROPÉEN**

(45) Date de publication du fascicule du brevet:
12.07.89

(21) Numéro de dépôt: **86400167.2**

(22) Date de dépôt: **28.01.86**

(51) Int. Cl.⁴: **A 61 M 7/00,** A 61 M 37/02,
A 61 D 7/02

(54) **Sonde pour la récolte d'embryons notamment sur les juments.**

(30) Priorité: **04.02.85 FR 8501494**

(43) Date de publication de la demande:
**03.09.86 Bulletin 86/36**

(45) Mention de la délivrance du brevet:
**12.07.89 Bulletin 89/28**

(84) Etats contractants désignés:
**AT BE CH DE GB IT LI LU NL SE**

(56) Documents cité:
**BE-A-379 330**
**BE-A-689 333**
**FR-A-2 091 058**
**FR-A-2 311 527**
**FR-A-2 340 078**
**FR-A-2 371 933**
**FR-M-2 284 312**

**M. SZYCHER: "Biocompatible polymers, metals and composites", 1983, pages 1036-1037, Lancaster, Technomic, US;**

(73) Titulaire: **Cassou, Robert, Rue Clémenceau, F-61300 L'Aigle (FR)**
Titulaire: **Cassou, Bertrand, Saint Symphorien des Bruyeres, F-61300 L'Aigle (FR)**
Titulaire: **Cassou, Maurice, Rue Clémenceau, F-61300 L'Aigle (FR)**

(72) Inventeur: **Cassou, Robert, Rue Clémenceau, F-61300 L'Aigle (FR)**
Inventeur: **Cassou, Bertrand, Saint Symphorien des Bruyeres, F-61300 L'Aigle (FR)**
Inventeur: **Cassou, Maurice, Rue Clémenceau, F-61300 L'Aigle (FR)**

(74) Mandataire: **Rodhain, Claude, Cabinet Claude Rodhain 30, rue la Boétie, F-75008 Paris (FR)**

LIBER, STOCKHOLM 1989

## Description

L'invention concerne une sonde pour la récolte d'embryons notamment sur les juments, du type constitué d'une canule pourvue sur sa périphérie, à distance de son extrémité introductive, d'un ballonet gonflable alimenté en fluide par un conduit capillaire, la portion operationnelle de canule comprise entre ce ballonet et cette extrémité comportant au moins un orifice de récupération du ou des embryons débouchant dans le canul intérieur de la canule. Une sonde de ce type et déjà connue par la demande de brevet français publiée sous le n° 2 340 078. Ces sondes sont généralement constituées d'une canule pourvue d'au moins un orifice de récupération des embryons, raccordée à une capacité contenant un milieu nourricier permettant de remplir les cornes utérines de l'animal pour assurer l'entraînement par immersion de l'embryon.

De telles sondes pour la récolte d'embryons de grandes dimensions (environ 1 mm à 7 jours) existent déjà, notamment pour la jument. Il faut toutefois observer que celles-ci sont mal adaptées à cette opération, en raison d'une part, de la nature même de la matière constituant la canule et, d'autre part, de la disposition et de la forme de l'orifice de récupération de l'embryon. En effet, les sondes connues pour ce type de prélèvement sont jusqu'alors constituées d'un simple tube de caoutchouc ayant un faible coefficient de glissement, et l'extrémité introductive de cette canule ne comporte qu'une seule ouverture radiale, sa section terminale étant en outre obturée.

La position et la forme de cette ouverture radiale rendent ainsi la récupération de l'embryon très aléatoire même lorsque les deux cornes utérines de l'animal ont été totalement immergées.

L'invention concerne donc une sonde du type précité, présentant l'originalité, de présenter une structure propre à permettre une récupération dans 90 % des cas de l'ovule fécondé.

L'invention concerne donc une sonde pour la récolte d'embryons, notamment sur les juments, du type constitué d'une canule pourvue sur sa périphérie, à distance de son extrémité introductive, d'un ballonnet gonflable alimenté en fluide par un conduit capillaire, la portion opérationnelle de canule comprise entre ce ballonnet et cette extrémité comportant au moins un orifice de récupération du ou des embryons débouchant dans le canal intérieur de la canule, sonde caractérisée en ce que ladite canule comporte une série d'orifices se succédant sur toute la longueur de la portion opérationnelle comprise entre ledit ballonnet et ladite extrémité introductive, décalés angulairement les uns par rapport aux autres dans une disposition en hélice pour intéresser l'intégralité de la périphérie de cette portion, chaque orifice étant en forme d'entonnoir pour canaliser les embryons en direction du canal intérieur de la canule.

La forme et la disposition en hélice des orifices permettent ainsi de récupérer les embryons sur 360° et de les canaliser, grâce à la forme rétrécie des orifices qui sont sensiblement plus larges à leur entrée afin de constituer des goulots de guidage, cette forme étant obtenue par un chanfrein pratiqué dans l'épaisseur de la matière constituant la canule proprement dite.

Selon une disposition avantageuse de l'invention, les orifices de récupération des embryons sont décalés angulairement de 90° selon un arrangement hélicoïdal.

D'autres caractéristiques et avantages de la sonde selon l'invention ressortiront de la description ci-après et des dessins annexés dans lesquels:

- la fig. 1 est une vue en coupe longitudinale tronquée de la sonde selon l'invention,
- la fig. 2 est une vue schématique illustrant la mise en place de ladite sonde.

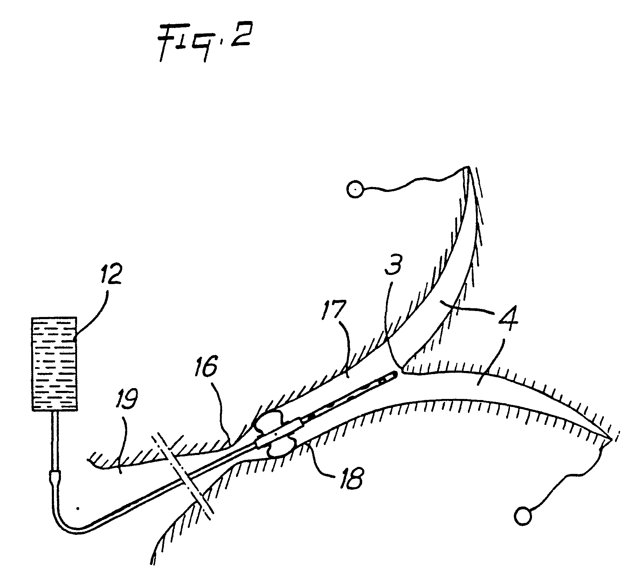

La sonde de récupération selon l'invention se présente, à l'instar des sondes connues, sous la forme d'une canule 1 suffisamment longue pour pouvoir franchir le col de l'utérus de manière à ce que son extrémité introductive $1_1$ puisse venir se positionner en regard de la zone de bifurcation 3 des deux cornes utérines 4 (Fig. 2).

Cette canule est de préférence réalisée à partir d'un tube en matière plastique extrudée, par exemple du PVC de type alimentaire, choisi non seulement pour sa neutralité, mais également pour son bon coefficient de glissement (en d'autres termes son faible coefficient de frottement) permettant une introduction et un retrait aisés et aussi sa semi-rigidité. Le diamètre extérieur de la canule est volontairement important (environ 10 mm) de façon à définir un canal intérieur 5 de section relativement importante (diamètre de plusieurs millimètres, supérieur aux dimensions usuelles d'un embryon de jument) de manière à permettre une bonne récupération des ovules fécondés, sans craindre la destruction partielle ou totale de leur enveloppe pellucide.

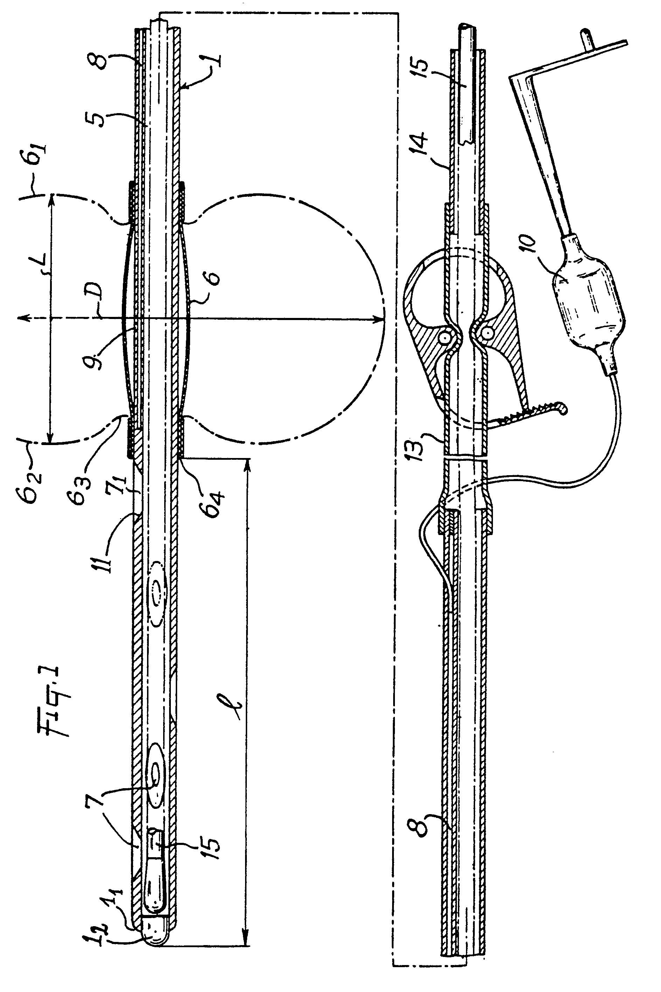

Cette canule comporte un ballonnet gonflable 6, connu en soi, mais présentant la particularité d'avoir une forme conçue pour faciliter la récolte des embryons et d'être positionné sur la canule à un endroit précis pour favoriser là encore le guidage des embryons en direction du canal intérieur 5 de la canule. On observrve en effet, sur l'illustration en trait mixte $6_1$ (qui est donnée en figure 1) que ce ballonnet, lorsqu'il est gonflé, a la forme, en section transversale, d'une mandarine, son diamètre D étant effectivement supérieur à sa largeur L en direction axiale, les flancs $6_2$ dudit ballonnet gonflé formant des joues proéminentes en oméga définissant une sorte de rampe $6_3$, du type "toboggan", orientant l'embryon en direction de l'extrémité introductive $1_1$ de la canule.

Avantageusement, le bord avant $6_4$ du ballonnet est situé à proximité immédiate (pratiquement au droit) du premier orifice $7_1$ de récupération des embryons de manière à les canaliser en direction de cet orifice. Le ballonnet est collé et ligaturé sur la canule en un endroit prédéterminé, selon le type ou la nature de l'animal, et est alimenté en fluide comprimé par un conduit capillaire 8 logé dans l'épaisseur de la canule 1, ce conduit débouchant par un ou plusieurs orifices 9 dans le volume interne du ballonnet. L'extrémité arrière du conduit capillaire 8 est reliée, de façon connue, à une capacité témoin 10 qui se gonfle lorsque le ballonnet 6 est correctement gonflé.

Les orifices 7 de récupération des embryons se succèdent et sont espacés sur toute la longueur "l" dite "portion opérationnelle" comprise entre le ballonnet 6 et l'extrémité introductive $1_1$ de la canule. Ces orifices présentent la particularité d'être décalés angulairement les uns par rapport aux autres, par exemple de 90°, dans une disposition en hélice de manière à intéresser l'intégralité de la périphérie de cette portion opérationnelle. Ainsi la communication entre la cavité utérine et le canal interne 5 de la canule peut se faire sur 360°.

Selon une autre particularité importante de l'invention, les orifices 7 de récupération ont une forme d'entonnoir de manière à participer au guidage de l'embryon dans le canal 5 de récupération. Cette forme d'entonnoir est obtenue par un chanfrein 11 pratiqué dans la matière, c'est-à-dire dans l'épaisseur de la canule. Enfin, ces orifices sont ovalisés pour accroître encore leur surface spécifique. On peut, bien entendu, conserver ouverte la section terminale de la canule 1 ou encore, comme illustré en figure 1, l'obturer à l'aide d'un bouchon amovible 12 constituant l'extrémité introductive de la sonde.

Cette sonde est reliée, au moment de son emploi, à une capacité 12 (fig. 2) contenant un milieu nourricier tel que du phosphate de saline tamponné (phosphate buffered saline: P.B.S.). La liaison entre la sonde et cette capacité 12 pouvant être réalisée d'au moins deux façons distinctes:

- selon la première forme de réalisation, la partie terminale arrière de la canule 1 est réunie à un raccord souple 13, facilement pinçable, lui-même accouplé à un embout-raccord 14, de type rigide, prolongé par un long tuyau souple (1 m ou 1,5 m) amovible. C'est l'extrémité de ce long tuyau souple qui est raccordée à la capacité 12 placée en hauteur pour obtenir une certaine pression.

- selon une seconde forme de réalisation, il est possible de supprimer les raccords 13 et 14 ainsi que le tuyau souple en téflon et de réaliser sous la forme monobloc une sonde semi-rigide se prolongeant au-delà du conduit capillaire 8, d'une longueur supérieure à celle introduite dans la capacité vaginale de l'animal, par exemple de plus de 2 m de long, directement reliée à la capacité 12. L'avantage de cette formule est d'obtenir une sonde présentant un canal interne 5 continu sur toute sa longueur et ne présentant aucune aspérité, rétrécissement ou saillie susceptible d'endommager ou de perdre l'enveloppe pellucide de l'embryon. En outre, la nature même de la matière constituant la canule s'oppose aux plis, goulots ou étranglements pouvant endommager l'embryon.

La mise en place de la sonde selon l'invention est d'une grande simplicité. On peut, pour parfaire sa rigidité et par conséquent favoriser son introduction, glisser à l'intérieur du canal interne 5 un mandrin 15 amovible se présentant sous la forme d'une longue tige métallique. On procède ensuite à l'introduction de la sonde dans la cavité utérine de l'animal de manière à amener l'extrémité $1_1$ de la canule en regard de la bifurcation 3 des deux cornes utérines 4, après que ladite canule ait franchi le col de l'utérus. Lorsque la canule est correctement positionnée, on procède à son immobilisation axiale et à l'obturation de la cavité utérine 17 par gonflage du ballonnet 6 en aval du col 16 de l'utérus. Ce ballonnet vient alors s'appliquer sur la paroi utérine 18 afin de masquer totalement l'orifice de sortie de l'utérus de manière à éviter que l'embryon puisse être entraîné hors de l'utérus et soit ainsi perdu.

Lorsque la cavité utérine est totalement obturée, on immerge les deux cornes 4 en déversant dans cette double cavité, le milieu nourricier contenu dans la capacité 12. Ce liquide vient remplir par simple gravité, les deux cornes utérines 4, immergeant ainsi totalement l'extrémité opérationnelle de la canule.

Il suffit ensuite de récupérer le liquide nourricier et de "piéger" l'embryon afin qu'il soit évacué avec ce liquide qui lui sert alors de véhicule. La récupération du milieu nourricier s'effectue simplement en débranchant l'extrémité arrière de la canule de la capacité 12 et en la reliant à un autre réservoir que l'on basculera ensuite progressivement vers le bas de manière à ce que le liquide s'écoule par simple gravité dans ce second réservoir. Cette opération est d'ailleurs facilitée par la forme inclinée de la cavité utérine de l'animal.

On recommencera l'opération d'immersion des cornes utérines une ou plusieurs fois, selon le type d'animal, en injectant dans la cavité de l'animal 300 cm³ de P.B.S. afin d'être certain d'avoir récupéré réellement l'embryon. Cette opération de récolte sera effectuée de préférence 7 jours après la fécondation de l'ovule et l'embryon ainsi récupéré pourra être, s'il est viable, soit mis en culture dans un milieu comparable à son environnement naturel, soit congelé par cryogénie, soit encore transféré immédiatement dans une mère porteuse. On pourra bien entendu, dans des conditions de température et de temps prédéterminées, récolter sur un même animal plusieurs embryons grâce à la forme et à la disposition originale des orifices de récupération.

## Revendications

1. Sonde pour la récolte d'embryons, notamment sur les juments, du type constitué d'une canule (1) pourvue sur sa périphérie à distance de son extrémité introductive, d'un ballonnet gonflable (6) alimenté en fluide par un conduit capillaire (8), la portion opérationnelle (I) de canule comprise entre ce ballonnet (6) et cette extrémité comportant au moins un orifice de récupération (7) du ou des embryons débouchant dans le canal intérieur (5) de la canule (1), sonde caractérisée en ce que ladite canule (1) comporte une série d'orifices (7) se succédant sur toute la longueur de la portion opérationnelle (I) comprise entre son ballonnet (6) et son extrémité introductive ($1_1$) , et décalés angulairement les uns par rapport aux autres dans une disposition en hélice pour intéresser l'intégralité de la périphérie de cette portion chaque orifice (6) étant en forme d'entonnoir pour canaliser les embryons en direction du canal intérieur (5) de la canule (1).

2. Sonde pour la récolte d'embryons selon la revendication 1, caractérisée en ce que le diamètre du canal interieur (5) de la canule est de plusieurs millimètres, les orifices (6) de récupération d'embryons étant de forme sensiblement ovale, l'entrée biseautée étant réalisée par un chanfrein (11) pratiqué dans l'épaisseur de la matière constituant ladite canule (1).

3. Sonde pour la récolte d'embryons selon l'une quelconque des revendications 1 et 2, caractérisée en ce que la section de l'extrémité introductive de la canule est obturée par un bouchon amovible ($1_2$).

4. Sonde pour la récolte d'embryons selon la revendication 1, caractérisée en ce que le ballonnet (6) est collé et ligaturé sur la canule (1), le bord avant ($6_4$) de ce ballonnet, dirigé vers l'extrémité introductive ($1_1$) de ladite canule, étant situé pratiquement au droit du premier orifice ($7_1$) de récupération des embryons.

5. Sonde pour la récolte d'embryons selon la revendication 4, caractérisée en ce que le ballonnet (6) a une dimension axiale inférieure à son diamètre, le gonflage lui conférant, en coupe longitudinale, une forme d'oméga définissant une rampe ($6_3$) du type "toboggan" guidant les embryons en direction du premier orifice ($7_1$) de récupération des embryons.

6. Sonde pour la récolte d'embryons, selon la revendication 1, caractérisée en ce que l'extrémité arrière de la canule est raccordée à un raccord souple (13), facilement obturable par pinçage, lui-même prolongé par unembout-raccord rigide (14) relié à un tuyau souple fixé de façon amovible à une capacité (12) contenant un milieu nourricier.

7. Sonde pour la récolte d'embryons selon la revendication 1, caractérisée en ce que la canule est d'une seule pièce et se prolonge au-delà du conduit capillaire (8) de gonflage du ballonnet (6) d'une longueur supérieure à celle introduite dans la cavité vaginale de l'animal.

8. Sonde pour la récolte d'embryons selon l'une quelconque des revendications 1 à 7, caractérisée en ce que la canule (1) est en matière plastique semi-rigide glissante, en particulier en PVC neutre du type alimentaire.

9. Sonde pour la récolte d'embryons selon la revendication 7, caractérisée en ce que la canule est exempte de toute aspérité sur toute la longueur de son canal intérieur évitant une destruction totale ou partielle de l'enveloppe pellucide de l'embryon.

10. Sonde pour la récolte d'embryons selon l'une quelconque des revendications 1 à 9, caractérisée en ce que la canule (1) comporte un mandrin rigide amovible (15) facilitant sa mise en place.

## Patentansprüche

1. Sonde zur Entnahme von Embryonen, insbesondere bei Stuten, mit einer Kanüle (1), welche an ihrem dem einzuführenden Ende entgegengesetzten Ende mit einem aufblasbaren kleinen Ballon (6) versehen ist, der durch eine kapillare Leitung (8) mit einen Fluid gespeist ist, wobei der zwischen dem kleinen Ballon (6) und dem einzuführenden Ende gelegene Arbeitsabschnitt (1) der Kanüle wenigstens eine Öffnung (7) zur Entnahme des oder der Embryonen aufweist, welche in den Innenkanal (5) der Kanüle (1) einmündet,

dadurch gekennzeichnet, daß die Kanüle (1) eine Reihe von Öffnungen (7) aufweist, welche aufeinanderfolgend über die ganze Länge des Arbeitsabschnitts (1) zwischen dem kleinen Ballon (6) und dem einzuführenden Ende ($1_1$) verteilt sind, und welche in einer wendelförmigen Anordnung winklig gegeneinander versetzt sind um die Gesamtheit dieses Abschnitts ausnützen zu können, und daß jede Öffnung (7) die Form eines Trichters aufweist, um die Embryonen in Richtung des Innenkanals (5) der Kanüle (1) zu kanalisieren.

2. Sonde zur Entnahme von Embryonen nach Anspruch 1,

dadurch gekennzeichnet, daß der Durchmesser des Innenkanals (5) der Kanüle mehrere Millimeter beträgt, daß die Öffnungen (7) zur Entnahme der Embryonen eine im wesentlichen ovale Form aufweisen, und daß die abgeschrägte Eintrittsöffnung durch eine Keilschrägung (11) verwirklicht ist, welche in die Materialdicke der Kanüle (1) eingearbeitet ist.

3. Sonde zur Entnahme von Embryonen nach Anspruch 1 oder 2,

dadurch gekennzeichnet, daß der Querschnitt des einzuführenden Endes der Kanüle durch einen lösbaren Pfropfen ($1_2$) verschlossen ist.

4. Sonde zur Entnahme von Embryonen nach Anspruch 1,

dadurch gekennzeichnet, daß der kleine Ballon (6) auf die Kanüle (1) geklebt und gebunden ist und daß der gegen das einzuführende Ende ($1_1$) der Kanüle gerichtete vordere Rand ($6_4$) dieses

kleinen Ballons sich praktisch rechts der ersten Öffnung ($7_1$) zur Entnahme der Embryone befindet.

5. Sonde zur Entnahme von Embryonen nach Anspruch 4,

dadurch gekennzeichnet, daß der kleine Ballon (6) ein geringeres axiales Ausmaß besitzt als sein Durchmesser, und daß das Aufblasen ihm im Längsschnitt eine Omega-Form verleiht, welche eine Rampe ($6_3$) in der Art einer "Rutsche" definiert, die die Embryonen zu der ersten Öffnung ($7_1$) zur Entnahme der Embryonen leitet.

6. Sonde zur Entnahme von Embryonen nach Anspruch 1,

dadurch gekennzeichnet, daß das hintere Ende der Kanüle an ein flexibles Verbindungsstück (13) angeschlossen ist, welche in einfacher Weise durch Abklemmen verschließbar und durch ein steifes Ansatz-Verbindungsstück (14) verlängert ist, das wiederum an einen flexiblen Schlauch angeschlossen ist, welcher lösbar an einem ein Nährmittel enthaltenden Vorratsbehälter (12) befestigt ist.

7. Sonde zur Entnahme von Embryonen nach Anspruch 1,

dadurch gekennzeichnet, daß die Kanüle einstückig ausgebildet ist und sich über die kapillare Leitung (8) zum Aufblasen des kleinen Ballons (6) hinaus um ein größeres Längenstück verlängert, als jenes, welches in die Vaginalöffnung des Tieres eingeführt ist.

8. Sonde zur Entnahme von Embryonen nach einem der Ansprüche 1 bis 7,

dadurch gekennzeichnet, daß die Kanüle (1) aus halbsteifem glatten Kunststoff besteht, insbesondere aus neutralem, nahrungsmittelechtem PVC.

9. Sonde zur Entnahme von Embryonen nach Anspruch 7,

dadurch gekennzeichnet, daß die Kanüle über die ganze Länge ihres Innenkanals frei von Unebenheiten ist, wodurch eine vollständige oder teilweise Zerstörung der durchsichtigen Embryohülle vermieden wird.

10. Sonde zur Entnahme von Embryonen nach einem der Ansprüche 1 bis 9,

dadurch gekennzeichnet, daß die Kanüle (1) einen steifen bewegbaren Dorn (15) aufweist, der die Plazierung der Kanüle erleichtert.

**Claims**

1. Probe for collecting embryos, in particular from mares, of the type constituted by a cannula (1) which is provided on its periphery, at a distance from its introductive end, with a shall inflatable balloon (6) which is fed with fluid by a capillary duct (8), the operational portion (1) of the cannula comprised between this small balloon (6) and this end comprising at least one recovery orifice (7) for the embryo or the embryos, opening into the interior channel (5) of the cannula (1), probe characterised in that the said cannula (1) comprises a series of orifices (7) arranged successively over the entire length of the operational portion (I) comprised between its small balloon (6) and its introductive end ($1_1$) and offset angularly in relation to one another in a helical arrangement in order to favour the integrity of the periphery of this portion, each orifice (6) being funnel shaped in order to guide the embryos in the direction of the interior channel (5) of the cannula (1).

2. Probe for collecting embryos according to claim 1, characterised in that the diameter of the interior channel (5) of the cannula is several millimeters, the orifices (6) for the recovery of embryos being substantially of oval shape, the bevelled entry being constructed in the form of a chamfer (11) arranged in the thickness of the material from which the said cannula (1) is made.

3. Probe for collecting embryos according to any one of the claims 1 to 2, characterized in that the section of the introductive end of the cannula is closed by a removable stopper ($1_2$).

4. Probe for collecting embryos according to claim 1, characterised in that the small balloon (6) is glued and ligatured onto the cannula (1), the front edge ($6_4$) of this small balloon, directed towards the introductive end ($1_1$) of the said cannula, being situated practically at a right angle to the first orifice ($7_1$) for recovery of the embryos.

5. Probe for collecting embryos according to claim 4, characterized in that the small balloon (6) has an axial dimension less than its diameter, the inflation conferring upon it (in longitudinal section) an omega shape defining a slope ($6_3$) of the "toboggan" type, which guides the embryos in the direction of the first orifice ($7_1$) for recovery of the embryos.

6. Probe for collecting embryos according to claim 1, characterized in that the rear end of the cannula is connected to a flexible connecting element (13) which is easily closable by pinching and is itself prolonged by a rigid socket-type connecting member (14) connected to a flexible pipe which is fixed in detachable manner to a storage vessel (12) containing a nutrient medium.

7. Probe for collecting embryos according to claim 1, characterized in that the cannula is all in one piece and is prolonged beyond the capillary duct (8) for inflation of the small balloon (6), by a length greater than that which is introduced into the vaginal cavity of the animal.

8. Probe for collecting embryos according to any one of the claims 1 to 7, characterized in that the cannula (1) is made of slippery semi-rigid plastic material, in particular of neutral PVC of the alimentary type.

9. Probe for collecting embryos according to claim 7, characterized in that the cannula is devoid of all roughness over the entire length of its interior channel, in order to prevent total or partial destruction of the transparent envelope of the embryo.

10. Probe for collecting embryos according to any one of the claims 1 to 9, characterised in that the cannula (1) comprises a rigid detachable mandrel (15) which facilitates its setting into position.

*Fig.1*

Fig. 2